# EUROPEAN PATENT APPLICATION

(11) **EP 1 232 737 A2**
(43) Date of publication of application: **21.08.2002**
(21) Application number: 02003706.5
(22) Date of filing: 19.02.2002
(51) Int. Cl.: A61F 13/56

(54) **Fastening means for a garment to be used with a disposable absorbent article**

(30) Priority: 20.02.2001 SE 0100576; 20.02.2001 US 269355 P
(71) Applicant: SCA Hygiene Products AB, 405 03 Göteborg (SE); TYTEX A/S, 7430 Ikast (DK)
(72) Inventor: Carlbark, Olle, 41757 Göteborg (SE); Rönnberg, Peter, 43133 Mölndal (SE); Strannemalm, Kenneth, 44831 Floda (SE); Balslev Sörensen, Bettina, 8600 Silkeborg (DK)
(74) Representative: Hammond, Andrew David

(57) **Abstract**

A garment (20) for use in absorbing bodily wastes. The garment is provided with a fastening system for fastening the garment around the waist of a wearer. The fastening system includes a first fastening arrangement (36) having two outwardly facing edges (40; 42) forming an angle (α) therebetween. The angle (α) is from 45° to 85°, preferably from 50° to 80°, more preferably from 55° to 75° and most preferably from 60° to 70°.

## Description

### TECHNICAL FIELD:

The present invention relates to a garment for use in absorbing bodily wastes according to the preamble of claim 1 and to a garment for use in absorbing bodily wastes according to the preamble of claim 8.

### BACKGROUND OF THE INVENTION:

Garments for use in absorbing bodily wastes may be worn by young children, as well as incontinent persons. These garments are fastened about the waist of the wearer. Such garments may be generally divided into two types; namely, a first type consisting of disposable garments and a second type consisting of washable reusable garments. Garments of the first type are generally termed disposable diapers and these usually comprise an absorbent batt sandwiched between a liquid pervious topsheet and a liquid impervious backsheet. A disposable diaper is intended to be discarded after an insult has been detected or once its absorbent capacity has been reached. A washable reusable garment is generally intended to be used with a disposable absorbent insert. Such a garment is normally termed a diaper cover and the disposable insert is held in place against the crotch region of the body of the wearer by the diaper cover. The disposable insert is discarded after an insult while the diaper cover may be reused and laundered when necessary.

Various types of fastening systems are employed to secure garments of the two types identified above about the waist of the wearer. Such types of fastening systems are generally either mechanical, such as buttons and button holes, press studs and poppers or hook-and-loop systems, or adhesive. Irrespective of the type of fastening system, a requirement is that the garment be easy to fasten about the waist of the wearer while at the same time being adequately secure during use. Particularly since diaper covers which are intended to be worn by active adults may be subjected to considerable forces, the fastening system must be designed to function reliably even during strenuous activity.

One example of a diaper cover is described in JP-A-8131469. As can be gleaned from the drawing of this Japanese abstract, the diaper cover presents a front panel over which lateral extensions of the rear panel are drawn and fastened to the front panel via a fastening system. The fastening system seemingly comprises two relatively widely spaced-apart strips of fastening material on each lateral extension.

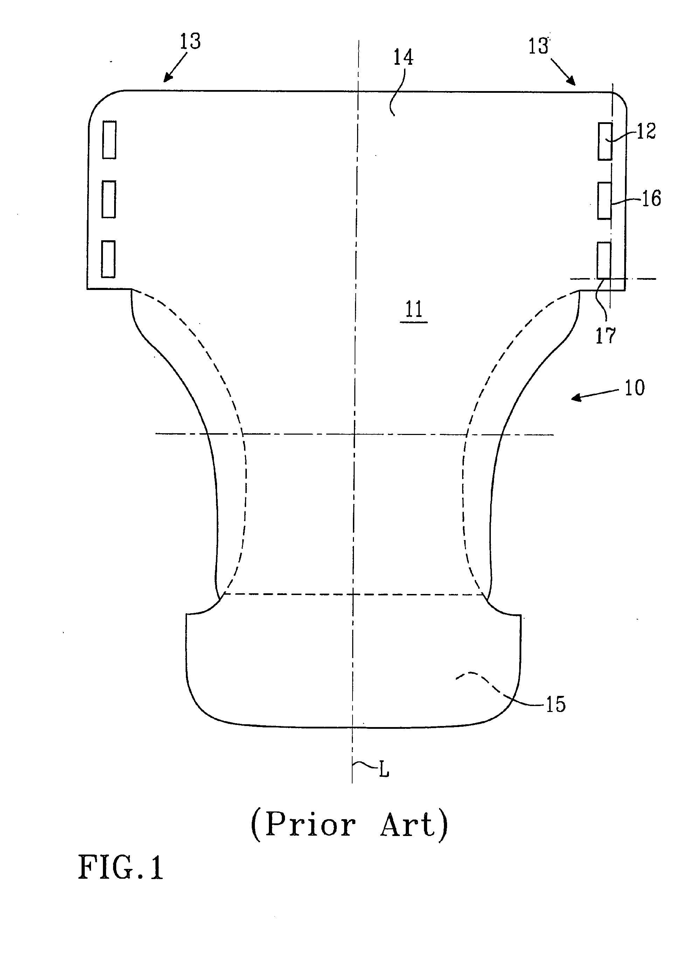

The present applicant has been manufacturing a range of diaper covers under the trade name Tena Cover. As illustrated in appended Fig. 1, the Tena Cover diaper cover 10 is provided on its surface 11 facing the wearer with a fastening system comprising a first fastening arrangement 12 located on each lateral extension 13 of the cover's rear panel 14. The first fastening arrangement comprises three patches of hook material positioned in a line substantially parallel to the longitudinal axis L of the diaper cover. The outer surface of the front panel 15 of the cover is provided with a region of loop material to which the patches of hook material can be fastened.

Although the fastening system of the Tena Cover has been shown to be adequately secure during use, it is deemed by some users to be somewhat inconvenient due to the fact that the patches of hook material are relatively widely spaced apart. This implies that it may be difficult to simultaneously engage all three patches with the loop material while maintaining the lateral extension of the diaper cover sufficiently tensioned in the longitudinal direction to prevent wrinkles or creases being formed. Thus, to prevent the formation of wrinkles or creases, the user must generally engage either the upper or the lower patch first and then sequentially engage the remaining patches while applying tension to the lateral extension in the direction of the longitudinal axis.

### SUMMARY OF THE INVENTION:

It is therefore an object of the present invention to provide a garment for use in absorbing bodily wastes, which garment is more convenient for the user to fasten, while still maintaining adequate security when fastened.

This object is achieved in accordance with the present invention by a garment as claimed in claim 1 and by a garment as claimed in claim 8.

Since, in accordance with the present invention, the outwardly facing edges of the first fastening arrangement form an acute angle, a more compact disposition of the fastening arrangement can be achieved without compromising fastening security. A more compact disposition implies that the region of the garment in which the fastening arrangement is disposed can be more easily grasped by one hand and applied to the complementary second fastening arrangement located at the end of the garment remote from the first fastening arrangement.

Advantageous embodiments of the garment according to the present invention are detailed in the dependent claims.

### BRIEF DESCRIPTION OF THE DRAWINGS:

The invention will be described in greater detail in the following by way of example only and with reference to embodiments shown in the attached drawings, in which:
Fig. 1 is a schematic plan view of a prior art garment for use in absorbing bodily wastes;
Fig. 2 is a schematic plan view of a first embodiment of a garment for use in absorbing bodily wastes according to the present invention, and
Fig. 3 is a schematic plan view of alternative designs for the first fastening system on a garment according to the present invention.

### DETAILED DESCRIPTION OF PREFERRED EMBODIMENTS:

Before describing preferred embodiments of the present invention, it may be helpful to consider in more detail the fastening arrangement of the prior art garment illustrated in Fig. 1.

Thus, the first fastening arrangement 12 presents a first outwardly facing edge 16 and a second outwardly facing edge 17. These edges are disposed at a right angle to each other. The outwardly facing edges are generally parallel to the peripheral edge of the garment and are located in close proximity to the peripheral edge to thereby ensure that there is a minimal amount of unsecured material along the outer margin of the lateral extension 13 of the rear panel 14. It will be apparent from Fig. 1 that the lateral extensions 13 have a considerable extension in the longitudinal direction, i.e. the portion of the peripheral edge adjacent the first outwardly facing edge 16 of the fastening arrangement typically has a length of between 15 cm and 20 cm.

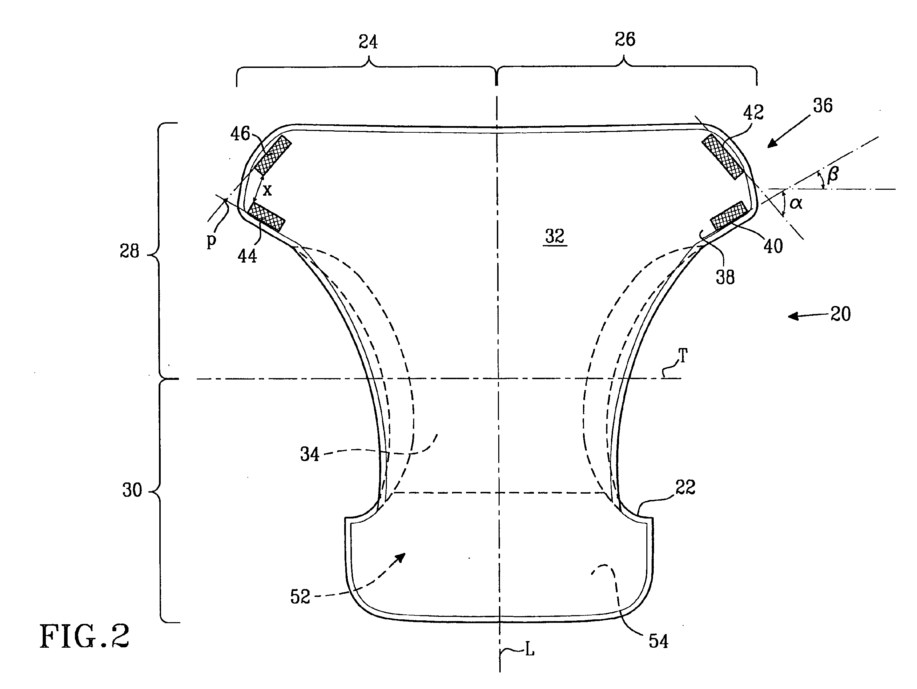

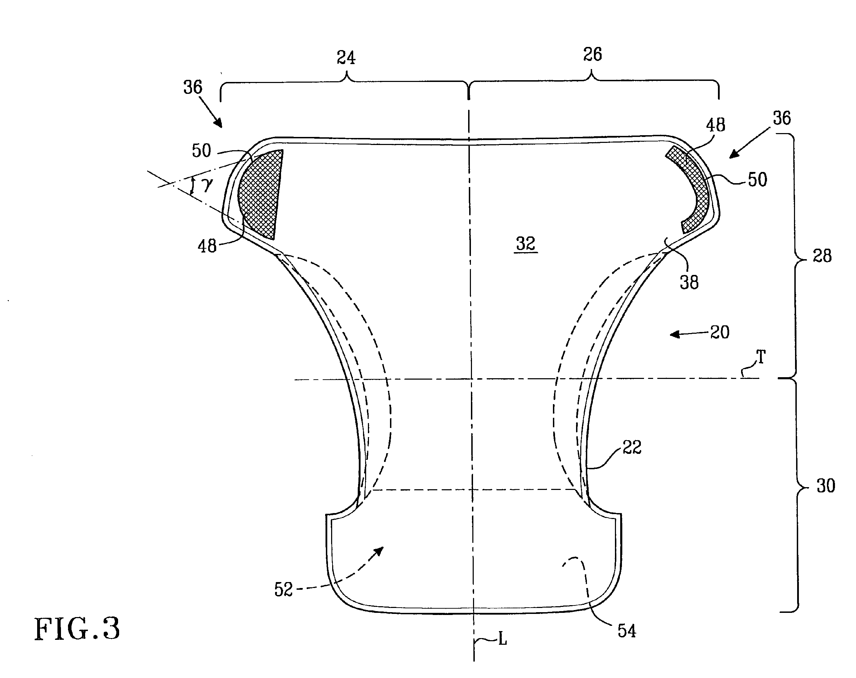

Turning now to Figs. 2 and 3, the garment of the present invention is generally denoted by reference numeral 20 and has a peripheral edge 22. A longitudinal axis L divides the garment 20 into a first lateral half 24 and a second lateral half 26. A transverse axis T perpendicular to longitudinal axis further divides the garment into a first end 28 and a second end 30. The garment 20 has an inwardly facing surface 32 which faces the wearer during use, and an outwardly facing surface 34 which faces away from the wearer during use. When the garment is in the form of a disposable diaper, the inwardly facing surface will thus be constituted by a liquid pervious facing sheet and the outwardly facing surface will be constituted by a liquid impervious backing sheet.

So that the garment may be secured around the waist of a wearer, the garment is provided with a fastening system. The fastening system may be either mechanical, such as buttons and button holes, press studs and poppers or hook-and-loop systems, or adhesive. As will be described in greater detail below, in a preferred embodiment of the present invention the fastening system comprises a hook-and loop arrangement. Irrespective of the actual type of fastening system, it comprises a first fastening arrangement, generally denoted by reference numeral 36, located on the first end 28 of the garment. More particularly, the first fastening arrangement 36 is located at an outer margin 38 of at least one of the first and the second lateral halves 24, 26. The outer margin 38 may be defined as that region of the garment which extends inwardly up to 5 cm from the peripheral edge 22 of the garment. The first fastening arrangement is to be regarded as being located at this outer margin if at least a portion of the first fastening arrangement is disposed within that region. As illustrated in Fig. 2, the first fastening arrangement 36 comprises two outwardly facing edges 40, 42 forming an angle α therebetween. The expression "outwardly facing edges" implies those edges which generally follow a portion of the peripheral edge 22 of the garment. Thus, a first one edge 40 of the outwardly facing edges faces towards the transverse axis T, while a second one edge 42 of the outwardly facing edges faces away from the transverse axis T. Advantageously, to avoid an excessive amount of unsecured material along the outer margin 38, the two outwardly facing edges 40, 42 of the first fastening arrangement should lie within 2.0 cm, preferably within 1.5 cm, of the peripheral edge 22 of the garment.

In accordance with the present invention, the angle α between the two outwardly facing edges 40, 42 is from 45° to 85°, preferably from 50° to 80°, more preferably from 55° to 75° and most preferably from 60° to 70°. By angling the outwardly facing edges 40, 42 in this manner, a more compact arrangement is attainable. Furthermore, by having the outwardly facing edges 40, 42 run substantially parallel to the peripheral edge 22 of the garment in the outer margin 38, the garment will taper towards a point in this region, thereby making it easier for a user to grasp the entire outer margin in one hand.

In a preferred embodiment of the invention, the first one edge 40 of the two outwardly facing edges subtends an angle β to the transverse axis T (or at least to a line parallel to the transverse axis T as illustrated) of from 0° to 50°, preferably from 15° to 40° and more preferably from 25 ° to 35 °. Without being bound to any theory, it is believed that by angling the first one edge 40 in this manner, at least a part of the first fastening arrangement may be aligned with those tensions which arise along the peripheral edge of the garment in the leg region, thereby being better able to accommodate these forces.

In Fig. 2, the first fastening arrangement 36 is shown as having two distinct patches 44, 46 of fastening material. It is to be understood, of course, that the invention may be implemented using a single patch having two outwardly facing edges angled in the manner described above. In the case where two or more patches of fastening material are utilized, the patches 44, 46 should be separated by a distance x of not more than 6.0 cm, preferably not more than 3.5 cm and most preferably not more than 2.0 cm. In other words, the closest distance between proximal points on adjacent patches should be no greater than the above recited distances.

Due to the provision of the angle α, virtual extensions of the two outwardly facing edges 40, 42 will intersect at a point P lying externally of the peripheral edge 22. Preferably, the point P is located at a distance of from 1.0 cm to 10.0 cm, more preferably from 2.5 cm to 5.0 cm, from the peripheral edge 22.

The advantages associated with the above-described embodiment may also be enjoyed by embodiments in which the first fastening arrangement presents a curved outwardly facing edge as opposed to two angled edges. Thus, Fig. 3 illustrates alternative embodiments of the present invention in which, for the sake of brevity, a garment is shown having a first shape of first fastening arrangement in the first lateral half 24 and a second shape of first fastening arrangement in the second lateral half 26. In both cases, the first fastening arrangement is illustrated as a single patch 48 of fastening material having an outwardly facing edge 50. The first shape is a segment of a circle while the second shape is of substantially constant width. In accordance with the invention, and irrespective of the actual shape, the outwardly facing edge 50 is curved such that there exists two points on the outwardly facing edge, tangents to which subtend an angle γ of not more than 90°, preferably not more than 60° and most preferably not more than 30°.

Advantageously, the first fastening arrangement of the illustrated embodiments comprises hook fastening material. The patches 44, 46 ,48 of hook material may comprise a base tape and monofilament hooks in 100% polyester having a weight of at least 320 g/m². Since the first fastening arrangement 36 preferably comprises a hook material, the fastening system must also comprise a second fastening arrangement, generally denoted by reference numeral 52, complementary to the first fastening arrangement. In the cases in which the garment is fastened around the waist of a wearer by fastening the first end 28 of the garment directly to the second end 30, the second fastening arrangement 52 is disposed on the second end 30 of the garment. Thus, the second fastening arrangement comprises at least one panel 54 of loop material. Such material may be 100% polyamide having a basis weight of about 210 g/m². Preferably, and as illustrated in the drawings, the panel 54 extends over substantially the entire width of the second end 30 of the garment 20. Alternatively, the second fastening arrangement 52 may consist of a plurality of panels of loop material suitably distributed over the second end 30 of the garment to thereby allow adjustment of the garment to different sizes of wearer.

For ease of fastening, the second end 30 of the garment may be adapted to cover a portion of the abdomen of a wearer. In other words, the first fastening arrangement 36 is disposed on the inwardly facing surface 32 of the garment on the end 32 which will cover the buttocks of the wearer during use and the panel 54 of loop material of the second fastening arrangement 52 is disposed on the outwardly facing surface 34 of the garment on the end 30 which covers a portion of the wearer's abdomen during use. Thus, to fasten the garment, the garment is drawn between the legs of the intended wearer, the second end 30 is held against the abdomen of the wearer and the regions of the first end 28 on which the first fastening arrangement 36 is disposed are drawn around the waist of the wearer and the first fastening arrangement 36 is made to engage the second fastening arrangement. Naturally, the fastening routine can be performed with the wearer either standing or lying on his/her back.

It is to be understood that the principles underlying the present invention may be implemented on both a disposable diaper and a diaper cover. It is further to be understood that the invention has been described above and illustrated in the drawings purely by way of example. Accordingly, the skilled person will appreciate that the present invention may be varied within the scope of the appended claims. For example, the relative positions of the patches of hook and loop material may be interchanged.

## Claims

1. A garment (20) for use in absorbing bodily wastes, said garment having:
a peripheral edge (22);
a longitudinal axis (L) dividing the garment into a first lateral half (24) and a second lateral half (26);
a transverse axis (T) perpendicular to said longitudinal axis, said transverse axis dividing the garment into a first end (28) and a second end (30);
a fastening system for fastening the garment around the waist of a wearer, the fastening system having a first fastening arrangement (36) located on said first end (28) of the garment, said first fastening arrangement being located at an outer margin (38) of at least one of said first and said second lateral halves (24; 26) and comprising two outwardly facing edges (40; 42) forming an angle (α) therebetween,
**characterized in that** said angle (α) is from 45° to 85°, preferably from 50° to 80°, more preferably from 55° to 75° and most preferably from 60° to 70°.

2. The garment (20) as claimed in claim 1, **characterized in that** a first one edge (40) of said two outwardly facing edges faces towards said transverse axis (T) and a second one edge (42) of said two outwardly facing edges faces away from said transverse axis, said first one edge (40) subtending an angle (β) to said transverse axis of from 0 ° to 50°, preferably from 15° to 40° and more preferably from 25° to 35°.

3. The garment (20) as claimed in claim 1 or 2, **characterized in that** said two outwardly facing edges (40, 42) run substantially parallel to the peripheral edge (22) of the garment in said outer margin (38).

4. The garment (20) as claimed in any one of the preceding claims, **characterized in that** said two outwardly facing edges (40, 42) lie within 2.0 cm, preferably within 1.5 cm, of the peripheral edge (22) of the garment.

5. The garment (20) as claimed in any one of the preceding claims, **characterized in that** said first fastening arrangement (36) comprises two distinct patches (44, 46) of fastening material.

6. The garment (20) as claimed in claim 5, **characterized in that** said patches (44, 46) of fastening material are separated by a distance (x) of not more than 6.0 cm, preferably not more than 3.5 cm and most preferably not more than 2.0 cm.

7. The garment (20) as claimed in any one of the preceding claims, **characterized in that** virtual extensions of said two outwardly facing edges (40, 42) intersect at a point (P) lying externally of said peripheral edge (22) at a distance of from 1.0 cm to 10.0 cm, preferably from 2.5 cm to 5.0 cm, from said peripheral edge.

8. A garment (20) for use in absorbing bodily wastes, said garment having:
a peripheral edge (22)
a longitudinal axis (L) dividing the garment into a first lateral half (24) and a second lateral half (26);
a transverse axis (T) perpendicular to said longitudinal axis, said transverse axis dividing the garment into a first end (28) and a second end (30);
a fastening system for fastening the garment around the waist of a wearer, the fastening system having a first fastening arrangement (36) located on said first end (28) of the garment, said first fastening arrangement being located at an outer margin (38) of at least one of said first and said second lateral halves and comprising an outwardly facing edge (50),
**characterized in that** said outwardly facing edge (50) is curved such that there exists two points on said outwardly facing edge, tangents to which subtend an angle (γ) of not more than 90°, preferably not more than 60° and most preferably not more than 30°.

9. The garment (20) as claimed in any one of the preceding claims, **characterized in that** said fastening system is a hook-and-loop fastening system.

10. The garment (20) as claimed in claim 9, **characterized in that** said first fastening arrangement (36) comprises hook fastening material.

11. The garment (20) as claimed in claim 10, **characterized in that** said fastening system comprises a second fastening arrangement (52) complementary to said first fastening arrangement, said second fastening arrangement being disposed on said second end (30) of the garment, said second fastening arrangement comprising at least one panel (54) of loop material such that said second fastening arrangement extends over substantially the entire width of said second end of said garment.

12. The garment (20) as claimed in any one of the preceding claims, **characterized in that** said second end (30) of said garment is adapted to cover a portion of the abdomen of a wearer.

13. The garment (20) as claimed in any one of the preceding claims, **characterized in that** said garment is a diaper cover.

14. The garment (20) as claimed in any one of claims 1 to 12, **characterized in that** said garment is a disposable diaper.

15. Use of an acutely angled fastening arrangement comprising hook material to fasten a garment around the waist of a wearer.
